# EUROPEAN PATENT APPLICATION

(11) **EP 2 201 895 A1**
(43) Date of publication of application: **30.06.2010**
(21) Application number: 09015902.1
(22) Date of filing: 22.12.2009
(51) Int. Cl.: A61B 6/03, G01T 1/164, G06T 11/00, G01T 1/161

(54) **Image diagnosis apparatus and image diagnosis method**

(30) Priority: 24.12.2008 JP 2008328027
(71) Applicant: Kabushiki Kaisha Toshiba, Minato-ku, Tokyo 105-8001 (JP); Toshiba Medical Systems Corporation, Otawara-shi Tochigi 324-8550 (JP)
(72) Inventor: Motomura, Nobutoku, Otawara-shi, Tochigi 324-8550 (JP); Kinda, Akiyoshi, Otawara-shi, Tochigi 324-8550 (JP)
(74) Representative: Kramer - Barske - Schmidtchen

(57) **Abstract**

A detector (25) detects gamma-rays emitted from inside an imaging region. An acquisition unit (26) acquires a plurality of first projection data sets associated with a plurality of projection angles via the detector (25). An attenuation-correction unit (83) attenuation-correct the plurality of first projection data sets based on a first CT image associated with the imaging region to generate a plurality of second projection data sets associated with the plurality of projection angles. An index calculation unit (85) calculates an index based on the plurality of second projection data sets. The index is corresponding to a degree of positional offset between the imaging region at the time of acquisition of a plurality of third projection data sets associated with the first CT image and the imaging region at the time of acquisition of the plurality of first projection data sets.

## Description

The present invention relates to an image diagnosis apparatus and image diagnosis method which perform image diagnosis by combining PET (Positron Emission Tomography) and X-ray CT (Computed Tomography).

There is available an image diagnosis apparatus (to be referred to as a PET-CT apparatus hereinafter) which performs image diagnosis by combining PET and X-ray CT. A PET-CT apparatus is an apparatus integrating a PET apparatus with an X-ray CT apparatus by using the same bed. The PET-CT apparatus is set to make a slice of a PET image physically match with a slice of a CT image.

The PET-CT apparatus, however, does not simultaneously obtain a PET image and a CT image. For this reason, the body movement or respiratory movement of a subject may cause an offset between a PET image and a CT image. Furthermore, the inaccuracy of apparatus installation or the like may cause a physical offset between a slice of a PET image and a slice of a CT image.

In order to prevent such positional offset, for example, a subject is fixed by, for example, lapping the body, or a breathing method is devised to reduce the respiratory movement. For example, CT is performed during a period in which the subject is expiring shallowly, whereas PET is performed during a period in which the subject is breathing freely (see, for example, Optical CT Breathing Protocol for Combined Thoracic PET/CT). In addition, physical positioning is performed by measuring a positional offset amount and shifting an image by image processing (linearly moving it in a three-dimensional space).

In some cases, the above methods cannot sufficiently prevent positional offsets.

Even if, for example, a subject is fixed by lapping the body, the part above the neck sometimes moves. Even if a respiration protocol with small positional offsets is used, it may not provide sufficient effect. Alternatively, it may not be possible for a subject to execute a required breathing method. Furthermore, the correction method which physically measures a positional offset amount may not be able to perform accurate positioning because of insufficient PET position resolution (about 5 mm) and the like.

As described above, although various methods have been provided to solve the problem of the positional offsets between PET images and CT images, the methods do not function effectively in some cases. In addition, there are neither means nor indexes for quantitative measurement of positional offsets between PET images and CT images.

It is an object of the present invention to provide an image diagnosis apparatus and image diagnosis method which can accurately correct the positional offset between a PET image and a CT image.

According to a first aspect of the present invention, an image diagnosis apparatus includes: a detector configured to detect gamma-rays emitted from inside an imaging region; an acquisition unit configured to acquire a plurality of first projection data sets associated with a plurality of projection angles via the detector; a correction unit configured to attenuation-correct the plurality of first projection data sets based on a first CT image associated with the imaging region to generate a plurality of second projection data sets associated with the plurality of projection angles; and a calculation unit configured to calculate an index based on the plurality of second projection data sets, the index being corresponding to a degree of positional offset between the imaging region at the time of acquisition of a plurality of third projection data sets associated with the first CT image and the imaging region at the time of acquisition of the plurality of first projection data sets.

According to a second aspect of the present invention, an image diagnosis method includes: causing a detector to detect gamma-rays emitted from inside an imaging region; causing an acquisition unit to acquire a plurality of first projection data sets associated with a plurality of projection angles via the detector; causing an attenuation correction unit to attenuation-correct each of the plurality of first projection data sets based on a first CT image associated with the imaging region to generate a plurality of second projection data sets associated with the plurality of projection angles; and causing a calculation unit to calculate an index based on the plurality of second projection data sets, the index being corresponding to a degree of positional offset between the imaging region at the time of acquisition of a plurality of third projection data sets associated with the first CT image and the imaging region at the time of acquisition of the plurality of first projection data sets.

The invention can be more fully understood from the following detailed description when taken in conjunction with the accompanying drawings, in which:
FIG. 1 is a side view schematically showing the outer appearance of an image diagnosis apparatus according to the first embodiment of the present invention;
FIG. 2 is a system block diagram of the image diagnosis apparatus in FIG. 1;
FIG. 3 is a flowchart showing a typical procedure for positional offset correction processing performed under the control of a system control unit in FIG. 1;
FIG. 4 is a view showing the positional offset between columnar phantoms in a case in which the positional offset between a PET image and a CT image is 8 mm;
FIG. 5 is a view showing the positional offset between columnar phantoms in a case in which the positional offset between a PET image and a CT image is 16 mm;
FIG. 6 is a view showing PET images in the case of FIG. 4;
FIG. 7 is a view showing PET images in the case of FIG. 5;
FIG. 8 is a graph showing the integral values (0th-order moments) of PET projection data sets in the case of FIG. 4;
FIG. 9 is a graph showing the integral values (0th-order moments) of PET projection data sets in the case of FIG. 5;
FIG. 10 is a view showing a human body phantom associated with a chest region;
FIG. 11 is a graph showing changes in the integral values (0th-order moments) of PET projection data sets as a function of projecting direction in the case of FIG. 10;
FIG. 12 is a block diagram showing the arrangement of an image diagnosis apparatus according to the second embodiment; and
FIG. 13 is a flowchart showing a typical procedure for positional offset correction processing performed under the control of a system control unit in FIG. 12.

The embodiments of the present invention will be described below with reference to the views of the accompanying drawing.

### (First Embodiment)

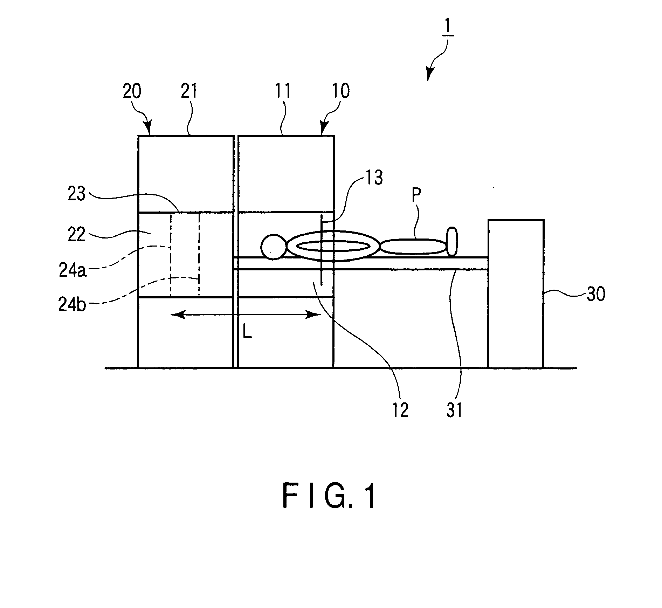

FIG. 1 is a side view schematically showing the outer appearance of an image diagnosis apparatus (PET-CT apparatus) 1 according to the first embodiment of the present invention.

As shown in FIG. 1, the image diagnosis apparatus 1 includes an X-ray CT (Computed Tomography) apparatus 10, a PET (Positron Emission Tomography) apparatus 20, and a bed apparatus 30.

The X-ray CT apparatus 10 is equipped with a CT gantry 11. The PET apparatus 20 is equipped with a PET gantry 21. The CT gantry 11 and the PET gantry 21 are arranged adjacent to each other, with a predetermined positional relationship between them being held, so as to be separably coupled to each other. A hollow portion 12 is formed in the CT gantry 11. A hollow portion 22 is formed in the PET gantry 21. The CT gantry 11 and the PET gantry 21 are arranged such that the center line of the hollow portion 12 almost coincides with the center line of the hollow portion 22.

The X-ray CT apparatus 10 and the PET apparatus 20 constituting the image diagnosis apparatus 1 have a common effective field of view.

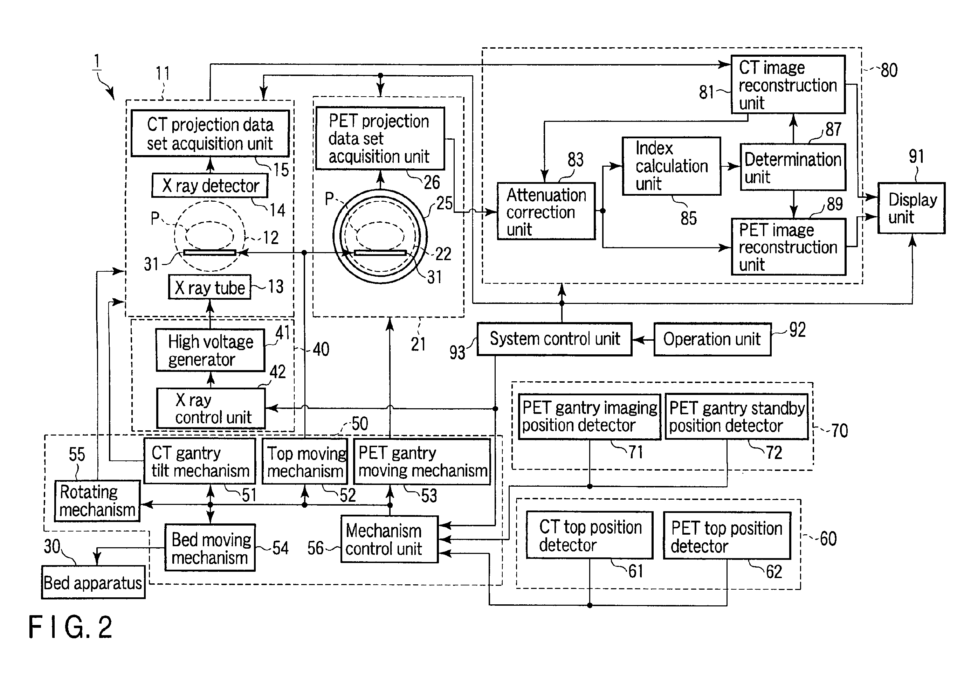

FIG. 2 is a system block diagram of the image diagnosis apparatus 1 in FIG. 1.

The image diagnosis apparatus 1 includes the CT gantry 11, the PET gantry 21, the bed apparatus 30, a high voltage generating unit 40, a mechanical unit 50, a top position detection unit 60, a PET gantry position detection unit 70, an image generating unit 80, a display unit 91, an operation unit 92, and a system control unit 93.

The CT gantry 11 performs CT of a subject P in an imaging region with X-rays. The CT gantry 11 includes an X-ray tube 13, an X-ray detector 14, and a CT projection data set acquisition unit 15. In addition, as described above, the CT gantry 11 has the hollow portion 12 having an almost cylindrical shape into which the subject P on a top 31 is transferred. The X-ray tube 13, the X-ray detector 14, and the CT projection data set acquisition unit 15 are held on a rotating ring (not shown) so as to be rotatable around the hollow portion 12. The CT gantry 11 is held by a holding unit (not shown) so as to be tiltable around a horizontal axis passing through the center of the hollow portion 12. The X-ray tube 13 and the X-ray detector 14 are arranged to face each other through the hollow portion 12. The X-ray tube 13 generates X-rays upon receiving a high voltage applied from a high voltage generating unit 40. The X-ray detector 14 detects X-rays transmitted through the subject P. The X-ray detector 14 generates an electrical signal corresponding the intensity of the detected X-rays. The CT projection data set acquisition unit 15 generates a projection data set associated with CT (to be referred to as a CT projection data set hereinafter) by performing preprocessing for the electrical signal generated by the X-ray detector 14. In this manner, the CT projection data set acquisition unit 15 acquires a plurality of CT projection data sets associated with a plurality of projection angles via the X-ray detector 14 during a CT period. The acquired CT projection data sets are supplied to the image generating unit 80.

The PET gantry 21 performs PET of the subject P with gamma-rays emitted from inside the subject P in the imaging region. More specifically, a drug (radio isotope) is administered into the subject P. The drug is labeled with a radio isotope that emits positrons. The positrons emitted from the drug are pair-annihilated with electrons to generate gamma-rays. The PET gantry 21 includes a detector 25 and a PET projection data set acquisition unit 26. As described above, the PET gantry 21 has the hollow portion 22 having an almost cylindrical shape into which the subject P on the top 31 is guided. The detector 25 is circumferentially arranged around the hollow portion 22. The detector 25 detects the gamma-rays emitted from inside the subject P and generates an electrical signal corresponding to the intensity of the detected gamma-rays. The PET projection data set acquisition unit 26 generates a projection data set associated with PET (to be referred to as a PET projection data set) by performing signal processing for the electrical signal generated by the detector 25. More specifically, the PET projection data set acquisition unit 26 generates a PET projection data set by performing position calculation processing, energy calculation processing, coincidence processing, preprocessing, and the like for the electrical signal from the detector 25. The preprocessing includes, for example, random correction and scattered radiation correction. In this manner, the PET projection data set acquisition unit 26 acquires a plurality of PET projection data sets associated with a plurality of projection angles via the detector 25 during a PET period. The acquired PET projection data sets are supplied to the image generating unit 80. Although the PET gantry 21 includes the PET projection data set acquisition unit 26, the first embodiment is not limited to this. For example, the PET projection data set acquisition unit 26 (especially, a constituent element to perform coincidence processing and a constituent element to perform preprocessing) can be provided outside the PET gantry 21 (for example, in a computer).

The CT gantry 11 and the PET gantry 21 are arranged such that the central axes of the hollow portion 12 and hollow portion 22 are lined up on the same straight line. Therefore, moving the subject P in one direction on the top 31 can make the subject P continuously pass through the hollow portion 12 and the hollow portion 22.

The bed apparatus 30 includes the top 31 on which the subject P is placed.

The high voltage generating unit 40 generates a high voltage necessary for X-ray application by the PET gantry 21. The high voltage generating unit 40 includes the high voltage generator 41 and an X-ray control unit 42. The high voltage generator 41 generates a high voltage between the anode and cathode of the X-ray tube 13 to accelerate thermal electrons generated from the cathode. The X-ray control unit 42 controls the high voltage generator 41 so as to adjust X-ray conditions such as a tube current, tube voltage, and application time in the X-ray tube 13 in accordance with an instruction from the system control unit 93.

The mechanical unit 50 tilts the CT gantry 11, moves the PET gantry 21, or moves the top 31. The mechanical unit 50 includes a CT gantry tilt mechanism 51, a top moving mechanism 52, a PET gantry moving mechanism 53, a bed moving mechanism 54, a rotating mechanism 55, and a mechanism control unit 56. The CT gantry tilt mechanism 51 tilts the CT gantry 11. The top moving mechanism 52 translates the top 31 in the vertical direction and the longitudinal direction. The PET gantry moving mechanism 53 moves the PET gantry 21 in the body axis direction of the subject P. The bed moving mechanism 54 moves the bed apparatus 30 to perform CT using the CT gantry 11 and PET using the PET gantry 21. The rotating mechanism 55 rotates the rotating ring provided on the CT gantry 11. The mechanism control unit 56 controls the CT gantry tilt mechanism 51, top moving mechanism 52, PET gantry moving mechanism 53, bed moving mechanism 54, and rotating mechanism 55.

It is possible to configure only one of the PET gantry 21 and the top 31 to be movable.

The top position detection unit 60 detects the position of the top 31 on the bed apparatus 30. The top position detection unit 60 includes a CT top position detector 61 and a PET top position detector 62. The CT top position detector 61 detects the position of the top 31 which moves from a PET position to a CT position for the execution of CT. The detected position of the top 31 is used to position the bed apparatus 30 for the execution of CT. The PET top position detector 62 detects the position of the top 31 which moves from the CT position to the PET position for the execution of PET. The detected position of the top 31 is used to position the bed apparatus 30 for the execution of PET.

The PET gantry position detection unit 70 detects the position of the PET gantry 21. The detection result obtained by the PET gantry position detection unit 70 is used to position the PET gantry 21. The PET gantry position detection unit 70 includes a PET gantry imaging position detector 71 and a PET gantry standby position detector 72. The PET gantry imaging position detector 71 detects the position of the PET gantry 21 which moves from the a standby position to an imaging position. The PET gantry standby position detector 72 detects the position of the PET gantry 21 which moves from the imaging position to the standby position.

The position signals detected by the top position detection unit 60 and the PET gantry position detection unit 70 are sent to the mechanism control unit 56 including a servo amplifier. The position signal from the top position detection unit 60 is used for control on the bed moving mechanism 54 by the mechanism control unit 56. The position signal from the PET gantry position detection unit 70 is used for control on the PET gantry moving mechanism 53 by the mechanism control unit 56.

The image generating unit 80 performs positional offset correction processing unique to this embodiment for a PET projection data set. This positional offset correction processing generates PET image data and CT image data whose positional offset has been corrected. This PET image data is supplied to the display unit 91. The image generating unit 80 calculates an index indicating the degree of positional offset between the imaging region at the time of acquisition of the CT projection data sets and an imaging region at the time of acquisition of the PET projection data sets. This index is referred to as a positional offset index hereinafter. The positional offset index data is supplied to the display unit 91.

More specifically, the image generating unit 80 includes a CT image reconstruction unit 81, an attenuation correction unit 83, an index calculation unit 85, a determination unit 87, and a PET image reconstruction unit 89.

The CT image reconstruction unit 81 reconstructs a CT image associated with a predetermined slice position based on a plurality of CT projection data sets associated with a plurality of projection angles. The CT image data is supplied to the attenuation correction unit 83 and the display unit 91.

The attenuation correction unit 83 performs attenuation correction for a plurality of PET projection data sets associated with the plurality of projection angles based on the CT image to generate a plurality of attenuation-corrected PET projection data sets associated with the plurality of projection angles. The plurality of attenuation-corrected PET projection data sets are supplied to the index calculation unit 85 and the PET image reconstruction unit 89.

The index calculation unit 85 calculates a positional offset index based on the plurality of attenuation-corrected PET projection data sets. The positional offset index represents the degree of positional offset between the imaging region at the time of acquisition of the plurality of CT projection data sets associated with the CT image used for attenuation correction and the imaging region at the time of acquisition of the PET projection data sets. In other words, the positional offset index represents the degree of positional offset between a reconstruction slice of the CT image and a reconstruction slice of the PET image. Assume that the category of positional offsets according to this embodiment includes both the positional offset of the subject in a reconstruction slice and the spatial positional offset of the reconstruction slice. The positional offset of the subject in the reconstruction slice originates from, for example, the respiratory movement of the subject. The spatial positional offset of a reconstruction slice originates from, for example, a positioning accuracy failure between the CT gantry 11 and the PET gantry 21. The positional offset index data is supplied to the determination unit 87.

The determination unit 87 determines whether the positional offset amount between a CT image and a PET image falls within an allowable range. In practice, the determination unit 87 determines whether the positional offset index is equal to or less than a threshold. Upon determining that the positional offset index is not equal to or less than the threshold, the determination unit 87 causes the CT image reconstruction unit 81 to shift the slice position of the CT image used for attenuation correction. Upon determining that the positional offset index is equal to or less than the threshold, the determination unit 87 causes the PET image reconstruction unit 89 to reconstruct a PET image.

The PET image reconstruction unit 89 reconstructs a PET image based on a plurality of attenuation-corrected projection data sets if the determination unit 87 determines that the positional offset index is equal to or less than the threshold. The positional offset amount between the slice position of the reconstructed PET image and the slice position of the CT image falls within the allowable range. As a consequence, the reconstructed PET image has undergone correction of the positional offset between itself and the CT image. The PET image data is supplied to the display unit 91.

The display unit 91 includes a monitor such as a liquid crystal display or a CRT. The display unit 91 superimposes and displays the CT image and the PET image from the image generating unit 80.

The operation unit 92 includes input devices such as switches, a keyboard, a trackball, a joystick, and a mouse. The operation unit 92 inputs subject information, an imaging position, a command, and the like via input devices in accordance with, for example, instructions from the operator. Subject information includes, for example, an age, sex, physique, examination region, examination method, and past examination history. Imaging conditions include, for example, an imaging target region (target organ), the tilt position of the CT gantry 11, the position of the PET gantry 21, and the position of the top 31.

The system control unit 93 systematically controls the respective units provided for the image diagnosis apparatus 1. For example, the system control unit 93 executes positional offset correction processing according to the first embodiment by controlling the respective units.

A concept concerning positional offset correction between a PET image and a CT image will be described below.

In a completely noise-free system, an attenuation-corrected PET projection data set satisfies Radon's theorem "the integral value (0th-order moment) of a projection data set is constant regardless of projection angle". On the other hand, a CT image is a visualization of the transmission coefficients of X-rays. Applying an appropriate transformation formula to a CT image makes it possible to use the resultant data for attenuation correction for a PET projection data set.

If the slice position of a PET image completely matches the slice position of a CT image, attenuation-corrected PET projection data sets satisfy Radon's theorem. That is, the 0th-order moments of the attenuation-corrected PET projection data are the same value at all projection angles. In other words, the 0th-order moments of attenuation-corrected projection data do not vary with changes in projection angle. If, however, there is a positional offset between a PET image and a CT image, an attenuation-corrected PET projection data sets do not satisfy Radon's theorem. That is, the 0th-order moments of the attenuation-corrected projection data sets vary with changes in projection angle. The image diagnosis apparatus 1 corrects the positional offset between the PET image and the CT image by using Radon's theorem described above.

The operation of the image diagnosis apparatus 1 according to the first embodiment will be described below. The general imaging operation of the X-ray CT apparatus 10 and PET apparatus 20 is well known, and hence a description of the operation will be omitted.

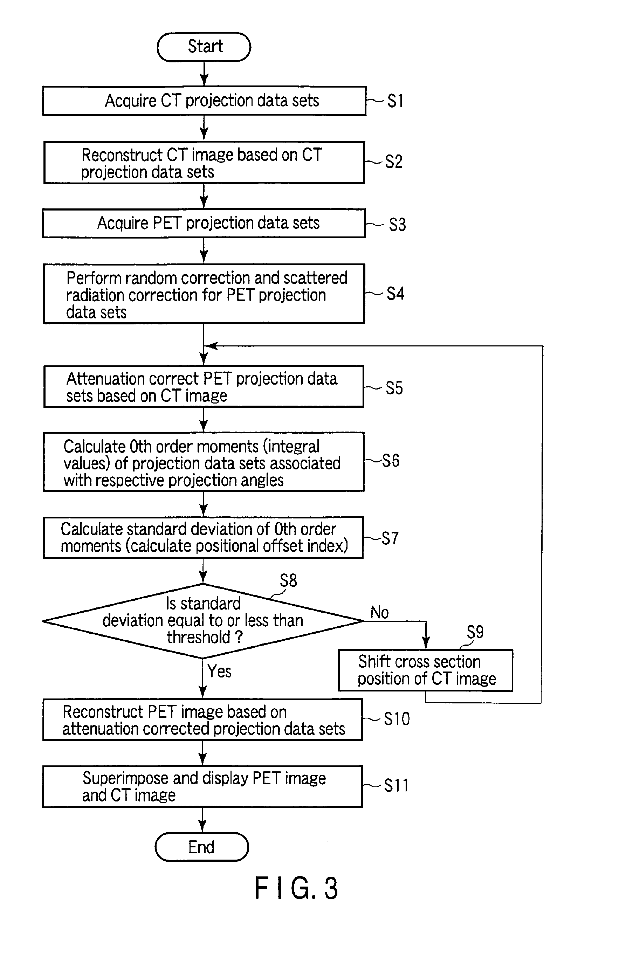

FIG. 3 is a flowchart showing a typical procedure for positional offset correction processing performed under the control of the system control unit 93 according to the first embodiment.

The system control unit 93 starts positional offset correction processing in response to a start request issued by an operator via the operation unit 92.

Upon starting positional offset correction processing, the system control unit 93 controls the CT apparatus 10 to perform CT. During CT, the system control unit 93 causes the CT projection data set acquisition unit 15 to perform acquisition processing (step S1). In step S1, the CT projection data set acquisition unit 15 acquires a plurality of CT projection data sets associated with a plurality of projection angles (step S1). The plurality of acquired CT projection data sets are supplied to the CT image reconstruction unit 81 and the display unit 91.

Upon performing step S1, the system control unit 93 causes the CT image reconstruction unit 81 to perform reconstruction processing (step S2). In step S2, the CT image reconstruction unit 81 reconstructs a CT image associated with a predetermined reconstruction slice based on the plurality of CT projection data sets. The reconstructed CT image is supplied to the attenuation correction unit 83.

Upon performing step S2, the system control unit 93 controls the PET apparatus 20 to perform PET. During PET, the system control unit 93 causes the PET projection data set acquisition unit 26 to perform acquisition processing (step S3). In step S3, the PET projection data set acquisition unit 26 acquires a plurality of PET projection data sets associated with a plurality of projection angles (step S3).

Upon performing step S3, the system control unit 93 causes the PET projection data set acquisition unit 26 to perform preprocessing (step S4). In step S4, the PET projection data set acquisition unit 26 performs preprocessing such as random correction and scattered radiation correction for the plurality of PET projection data sets. Such random correction and scattered radiation correction are performed for PET projection data sets to implement a noise-free system. Note that positional offset correction according to the first embodiment uses the 0th-order moments of PET projection data sets. It can therefore be assumed that the statistic noise has a small influence on positional offset correction. Assume that in this embodiment, preprocessing does not include attenuation correction. The plurality of preprocessed PET projection data sets are supplied to the attenuation correction unit 83.

The above operation on the CT apparatus 10 side (steps S1 and S2) and the operation on the PET apparatus 20 side (steps S3 and S4) are not limited to this sequence. For example, it is possible to perform the operation on the PET apparatus 20 side before the operation on the CT apparatus 10 side.

Upon performing step S4, the system control unit 93 causes the attenuation correction unit 83 to perform attenuation correction processing (step S5). In step S5, the attenuation correction unit 83 attenuation-corrects the plurality of PET projection data sets from the PET projection data set acquisition unit 26 based on the CT image from the CT image reconstruction unit 81. Performing attenuation correction will generate a plurality of attenuation-corrected PET projection data sets associated with a plurality of projection angles. The attenuation-corrected PET projection data sets are supplied to the index calculation unit 85 and the PET image reconstruction unit 89. Note that attenuation-corrected data is generated from a CT image. For this reason, the statistic noise of attenuation correction relative to positional offset correction is small.

Upon performing step S5, the system control unit 93 causes the index calculation unit 85 to perform 0th-order moment calculation processing (step S6). In step S6, the index calculation unit 85 calculates 0th-order moments by integrating the projection values of the attenuation-corrected PET projection data sets along spatial positions with respect to the respective projection angles. The index calculation unit 85 calculates 0th-order moments at all the projection angles. In step S6, therefore, the index calculation unit 85 calculates a plurality of 0th-order moments associated with a plurality of projection angles.

Upon performing step S6, the system control unit 93 causes the index calculation unit 85 to perform positional offset index calculation processing (step S7). In step S7, the index calculation unit 85 calculates a positional offset index associated with the CT image used in step S5 and the PET image based on the plurality of 0th-order moments. This PET image is an image reconstructed based on the plurality of PET projection data sets acquired in step S3. At the time of step S7, this PET image has not been reconstructed. A positional offset index is, for example, an index quantitatively indicating the degree of variation in 0th-order moment. More specifically, it is preferable to use, as a positional offset index, a standard deviation or variance as a statistic index indicating the degree of variation. Assume that a standard deviation will be used as a positional offset index for a concrete description. That is, the index calculation unit 85 calculates the standard deviation of a plurality of 0th-order moments. The standard deviation data is supplied to the determination unit 87.

Upon performing step S7, the system control unit 93 causes the determination unit 87 to perform determination processing (step S8). In step S8, in order to determine whether the positional offset amount between a reconstruction slice of the PET image and a reconstruction slice of the CT image falls within an allowable range, the determination unit 87 determines whether the standard deviation from the index calculation unit 85 is equal to or less than a threshold. In other words, the determination unit 87 determines whether the 0th-order moments indicate a variation equal to or more than the standard deviation. Note that the threshold is set in advance by the operator or the like via the operation unit 92. Upon determining that the standard deviation is not equal to or less than the threshold (the 0th-order moments indicate a variation equal to or more than the standard deviation), the determination unit 87 determines that the positional offset amount between the reconstruction slice of the PET image and the reconstruction slice of the CT image falls outside the allowable range. Upon determining that the standard deviation is equal to or less than the threshold (the 0th-order moments indicate a variation falling within the standard deviation), the determination unit 87 determines that the positional offset amount between the reconstruction slice of the PET image and the reconstruction slice of the CT image falls within the allowable range.

If it is determined that the standard deviation is not equal to or less than the threshold (step S8: NO), the system control unit 93 causes the CT image reconstruction unit 81 to perform shift processing for the reconstruction slice (step S9). In step S9, the CT image reconstruction unit 81 shifts the position of the reconstruction slice of the CT image used in step S5. More specifically, the CT image reconstruction unit 81 three-dimensionally moves or rotates the reconstruction slice. With this operation, based on the plurality of CT projection data sets, the CT image reconstruction unit 81 reconstructs a CT image whose reconstruction slice position is shifted. Thereafter, the system control unit 93 advances to step S5. In this manner, the processing from step S5 to step S9 is repeated until the standard deviation becomes equal to or less than the threshold.

If it is determined that the standard deviation is equal to or less than the threshold (step S8: YES), the system control unit 93 causes the PET image reconstruction unit 89 performs reconstruction processing (step S10). In step S10, the PET image reconstruction unit 89 reconstructs a PET image based on the plurality of attenuation-corrected PET projection data sets associated with a plurality of projection angles which are generated in step S5. With the determination processing in step S8, the positional offset amount between the PET image and the CT image falls within the allowable range. As a consequence, therefore, the reconstructed PET image has undergone correction of the positional offset between itself and the CT image. A PET image is reconstructed by using a standard deviation in this manner in consideration of a positional offset. The reconstructed PET image data is supplied to the display unit 91.

Upon performing step S10, the system control unit 93 causes the display unit 91 to perform fusion display processing (step S11). In step S11, the display unit 91 superimposes and displays the PET image reconstructed in step S10 and the CT image which almost coincides with the PET image. As described above, the PET image and the CT image, which are display targets, are positionally matched properly. A doctor or the like can therefore perform accurate image diagnosis by observing the superimposed images.

Upon performing step S11, the system control unit 93 terminates positional offset correction processing. As described above, in positional offset correction processing, an attenuation-corrected PET projection data set is generated every time the slice position of a CT image is shifted. A 0th-order moment and a standard deviation are then calculated. The calculated standard deviation is compared with a threshold. The reconstruction slice of the PET image is kept still during this processing. That is, positional offset correction processing corrects the positional offset between the slice position of a PET image and the slice position of a CT image by shifting the slice position of the CT image while fixing the slice position of the PET image.

Positional offset correction processing according to the first embodiment makes it possible to automatically correct the positional offset between a PET image and a CT image. That is, the operator need not perform any manual operation. Therefore, this positional offset correction processing can easily correct the positional offset between a PET image and a CT image without imposing any load on the operator. In addition, this positional offset correction processing can eliminate the subjectivity of the operator in positional offset correction because of the absence of manual operation by the operator. Accordingly, this positional offset correction processing can provide stable positional offset correction accuracy relative to that in manual operation. In addition, a positional offset index used for the positional offset correction processing is not calculated for every image processing for a PET image and a CT image but is calculated based on Radon's theorem which holds for PET projection data sets in a mathematically strict sense. This positional offset index therefore objectively and properly represents the degree of positional offset between an imaging region at the time of acquisition of PET projection data sets and an imaging region at the time of acquisition of CT projection data sets.

In step S8 in the above positional offset correction processing, the determination unit 87 compares the standard deviation with the threshold. However, positional offset correction processing is not limited to this. For example, it is possible to determine whether the standard deviation is the minimum value.

In the above positional offset correction processing, the system control unit 93 reconstructs a PET image based on attenuation-corrected PET projection data sets associated with a standard deviation equal to or less than the threshold. However, positional offset correction processing is not limited to this. For example, the system control unit 93 can calculate a plurality of standard deviations associated with a plurality of reconstruction slices and reconstruct a PET image based on an attenuation-corrected PET projection data set associated with the minimum value of the plurality of calculated standard deviations. This makes it possible to minimize the positional offset amount between the PET image and the CT image.

The following description concerns positional offset between a CT image and a PET image in a case in which columnar phantoms are exemplified.

A columnar phantom with a radio isotope (RI) being uniformly distributed in a 20-cm diameter columnar vessel will be exemplified. The following will show a noise-free numerical simulation result.

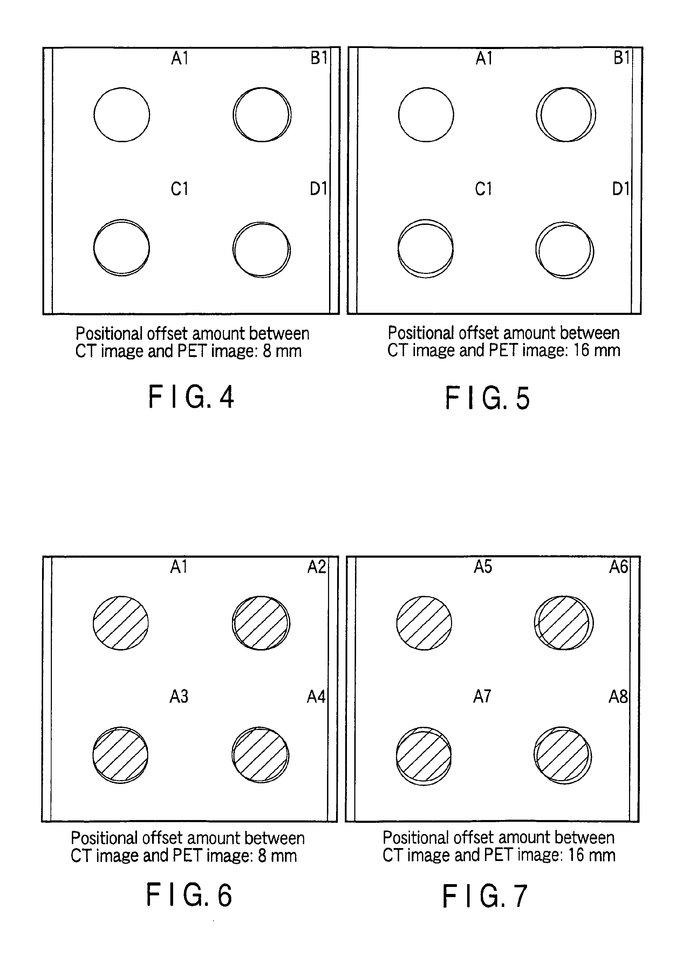

In a columnar phantom, a region in which an RI is distributed is identical to a region in which an attenuating element (water equivalent: 0.096 cm⁻¹) is distributed, which is a 20-cm diameter circle. FIG. 4 is a view showing the positional offset between columnar phantoms in a case in which the positional offset amount between a PET image and a CT image is 8 mm. Referring to FIG. 4, reference symbol A1 denotes a case without any positional offset; B1, a case with a positional offset amount of 8 mm in the X direction; C1, a case with a positional offset amount of 8 mm in the Y direction; and D1, a case with a positional offset amount of 8 mm in the X and Y directions. FIG. 5 is a view showing the positional offset between columnar phantoms in a case in which the positional offset between a PET image and a CT image is 16 mm. Referring to FIG. 5, reference symbol A1 denotes a case without any positional offset; B1, a case with a positional offset amount of 16 mm in the X direction; C1, a case with a positional offset amount of 16 mm in the Y direction; and D1, a case with a positional offset amount of 16 mm in the X and Y directions.

FIG. 6 is a view showing PET images in the case of FIG. 4. Reference numeral A1 in FIG. 6 denotes a PET image in a case in which there is no positional offset between the PET image and the CT image. Reference numeral B1 in FIG. 6 denotes a PET image in a case in which the positional offset amount between the PET image and the CT image is 8 mm in the X direction. Reference numeral C1 in FIG. 6 denotes a PET image in a case in which the positional offset amount between the PET image and the CT image is 8 mm in the Y direction. Reference numeral D1 in FIG. 6 denotes a PET image in a case in which the positional offset amount between the PET image and the CT image is 8 mm in the X and Y directions. FIG. 7 is a view showing PET images in the case of FIG. 5. Reference numeral A1 in FIG. 7 denotes a PET image in a case in which there is no positional offset between the PET image and the CT image. Reference numeral B1 in FIG. 7 denotes a PET image in a case in which the positional offset amount between the PET image and the CT image is 16 mm in the X direction. Reference numeral C1 in FIG. 7 denotes a PET image in a case in which the positional offset amount between the PET image and the CT image is 16 mm in the Y direction. Reference numeral D1 in FIG. 7 denotes a PET image in a case in which the positional offset amount between the PET image and the CT image is 16 mm in the X and Y directions. As shown in FIGS. 6 and 7, the positional offsets between PET images and CT images degrade uniform RI distributions.

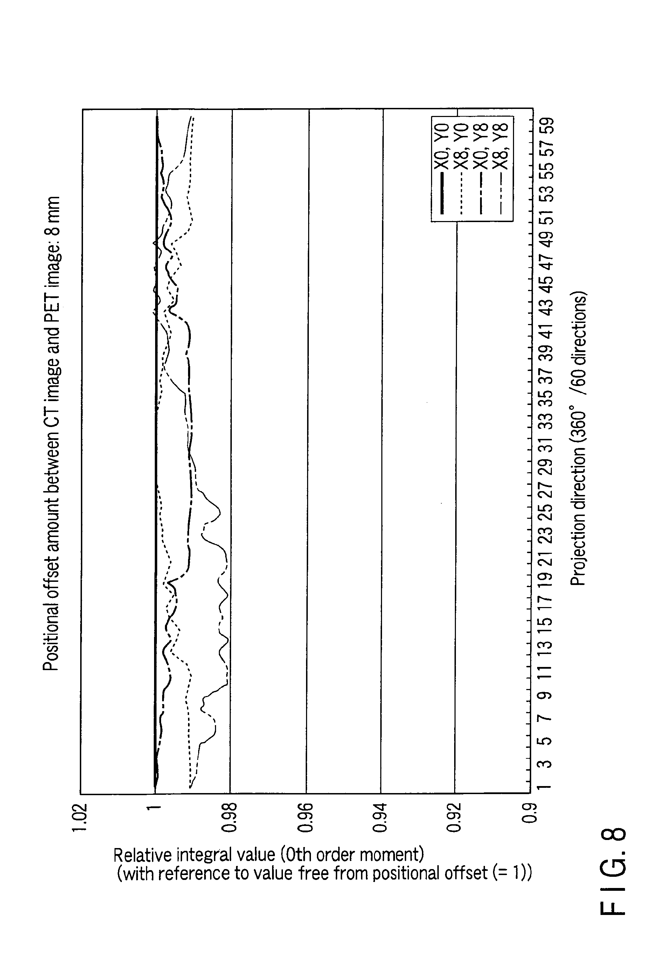

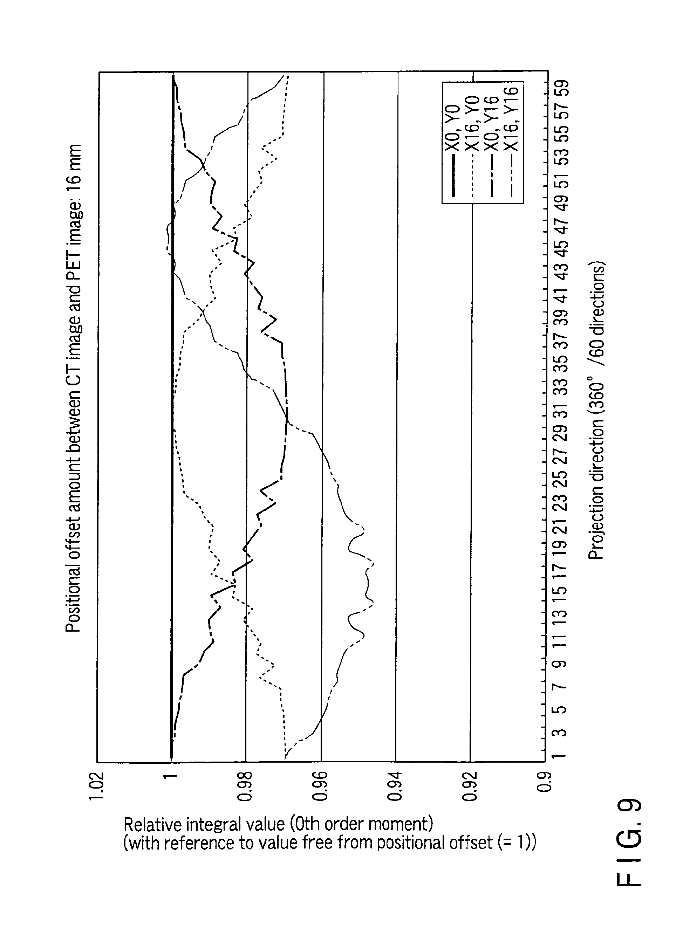

FIG. 8 is a graph showing 0th-order moments corresponding to projecting directions in the case of FIG. 4. FIG. 9 is a graph showing 0th-order moments corresponding to projecting directions in the case of FIG. 5. As shown in FIGS. 8 and 9, the positional offsets between PET images and CT images make 0th-order moments non-uniform depending on the projecting directions.

The following are examples of variations in 0th-order moment, expressed by standard deviations, with changes in projecting direction:

| | |
|---|---|
| (X, Y)= (0, 0) | :0.07% |
| (X, Y)= (8, 0) | :0.35% |
| (X, Y)= (0, 8) | :0.35% |
| (X, Y)= (8, 8) | :0.64% |
| (X, Y)= (16, 0) | :1.11% |
| (X, Y)= (0, 16) | :1.08% |
| (X, Y)= (16, 16) | :1.91% |

where (X, Y) represents a positional offset amount in mm.

For example, it is possible to correct the positional offset between a PET image and a CT image by searching for X and Y shift amounts which minimize the standard deviation of 0th-order moments.

It is possible to use, for example, a standard deviation as an index indicating the degree of positional offset between a PET image and a CT image. Correcting a position in a direction to minimize a standard deviation can therefore eliminate or minimize the positional offset between the PET image and the CT image.

The following will describe the positional offset between a CT image and a PET image by exemplifying a human body phantom.

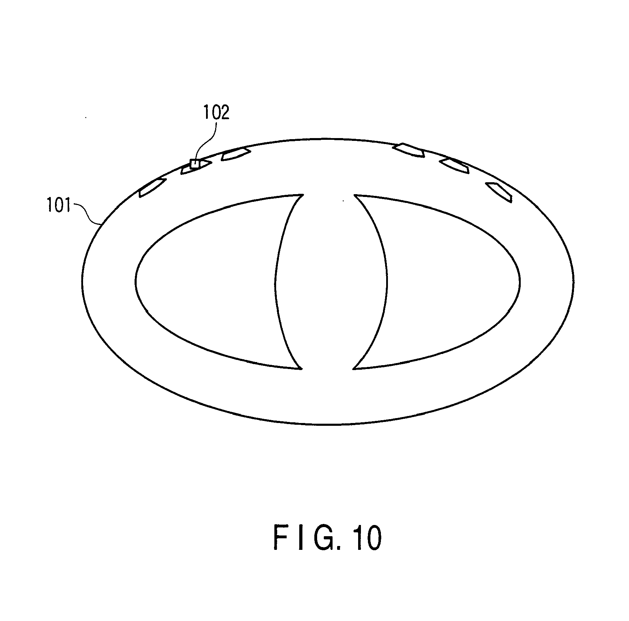

The following will exemplify a chest (lung) region in which a positional offset is likely to occur between a PET image and a CT image due to respiration. FIG. 10 is a view showing a human body phantom 101 associated with the chest region. As shown in FIG. 10, a 12 mm x 12 mm RI distribution (tumor) 102 is assumed in a costal region in the human body phantom 101.

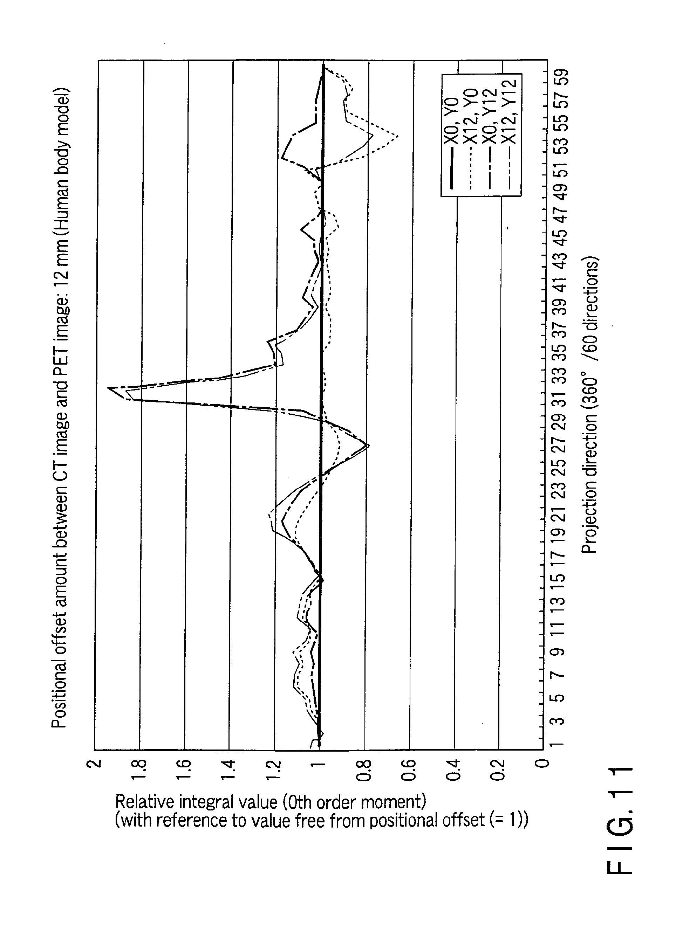

FIG. 11 is a graph showing changes in the integral values (0th-order moments) of PET projection data sets as a function of projecting direction in the case of FIG. 10. As shown in FIG. 11, owing to the positional offset between a PET image and a CT image, the 0th-order moment varies with changes in projecting direction. For example, the following are variations in 0th-order moment with changes in projecting direction, which are expressed by standard deviations.

| | |
|---|---|
| (X, Y)= (0, 0) | :0.91% |
| (X, Y)= (12, 0) | :8.36% |
| (X, Y)= (0, 12) | :15.80% |
| (X, Y)= (12, 12) | :16.60% |

where (X, Y) represents a positional offset amount in mm.

For example, it is possible to correct the positional offset between a PET image and a CT image by searching for X and Y shift amounts which minimize the standard deviation of 0th-order moments.

It is possible to use, for example, a standard deviation as an index indicating the degree of positional offset between a PET image and a CT image.

As described above, according to the first embodiment, it is possible to perform correction so as to minimize the positional offset between a PET image and a CT image by obtaining their positional relationship which minimizes variations in 0th-order moment with changes in projecting direction.

As described above, the image diagnosis apparatus and image diagnosis method according to the first embodiment can accurately correct the positional offset between a PET image and a CT image.

### (Second Embodiment)

The second embodiment of the present invention will be described next. Note that the same reference numerals as in the first embodiment denote constituent elements having almost the same functions in the second embodiment, and a repetitive description will be made only when required.

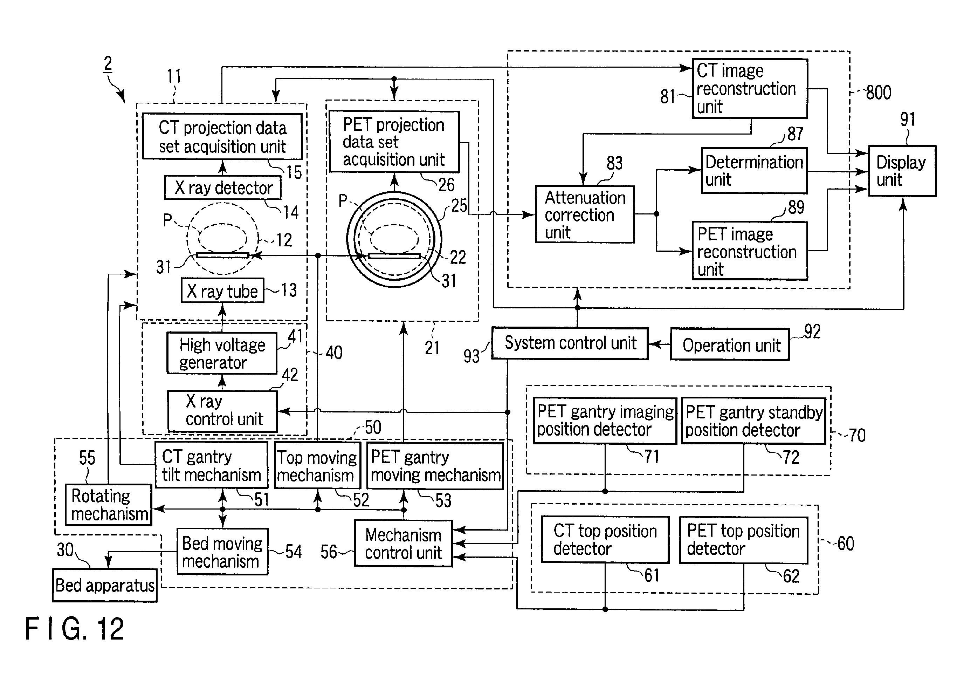

FIG. 12 is a system block diagram of an image diagnosis apparatus 2 according to the second embodiment. As shown in FIG. 12, an image generating unit 800 of the image diagnosis apparatus 2 includes a CT image reconstruction unit 81, an attenuation correction unit 83, an index calculation unit 85, and a PET image reconstruction unit 89. As is obvious from the comparison with FIG. 2, the image generating unit 800 according to the second embodiment does not include the determination unit 87 included in the image generating unit 80 according to the first embodiment.

The CT image reconstruction unit 81 reconstructs a CT image. The CT image data is supplied to the attenuation correction unit 83 and a display unit 91. The attenuation correction unit 83 generates a plurality of attenuation-corrected PET projection data sets associated with a plurality of projection angles based on the CT image. The generated attenuation-corrected PET projection data sets are supplied to the index calculation unit 85 and the PET image reconstruction unit 89. The index calculation unit 85 calculates a plurality of 0th-order moments associated with the plurality of projection angles by integrating the plurality of attenuation-corrected PET projection data sets. The index calculation unit 85 calculates a positional offset index (e.g., a standard deviation) based on the plurality of 0th-order moments. The calculated positional offset index data is supplied to the display unit 91. The PET image reconstruction unit 89 reconstructs a PET image based on the plurality of attenuation-corrected PET projection data sets. The PET image data is supplied to the display unit 91.

The display unit 91 superimposes and displays the CT image and the PET image. The display unit 91 also displays the positional offset index.

The first embodiment described above obtains the positional relationship between a PET image and a CT image, which minimizes variations in 0th-order moment with changes in projection angle (projecting direction), thereby obtaining a slice position at which the positional offset between them falls within the allowable range. In the second embodiment, a 0th-order moment variation is used as an index indicating the positional offset between a PET image and a CT image.

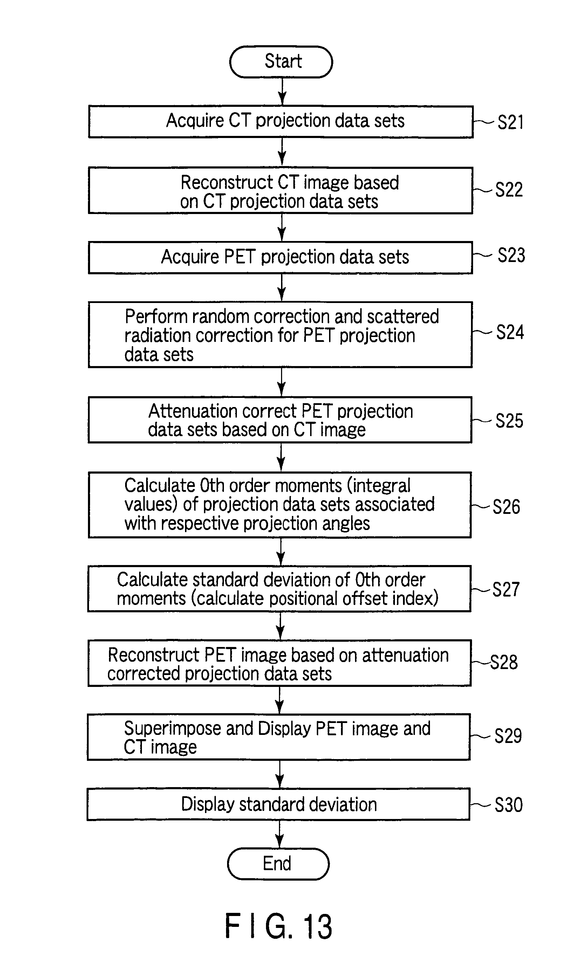

FIG. 13 is a flowchart showing a typical procedure for positional offset correction processing performed under the control of the system control unit 93 according to the first embodiment. The processing from step S21 to step S27 in FIG. 13 is the same as that from step S1 to step S7 in FIG. 3. A description of processing up to step S27 will therefore be omitted.

Upon calculating a standard deviation in step S27, the system control unit 93 causes the PET image reconstruction unit 89 to perform reconstruction processing (step S28). In step S28, the PET image reconstruction unit 89 reconstructs a PET image based on the plurality of attenuation-corrected PET projection data sets generated in step S25. The reconstructed PET image data is supplied to the display unit 91.

Upon performing step S28, the system control unit 93 causes the display unit 91 to perform fusion display processing (step S29). In step S29, the display unit 91 superimposes and displays the CT image reconstructed in step S22 and the PET image reconstructed in step S28.

Upon performing step S29, the system control unit 93 causes the display unit 91 to perform display processing for a positional offset index (step S30). In step S30, the display unit 91 displays the positional offset index calculated in step S27, e.g., a standard deviation. This standard deviation is displayed as an index indicating the degree of positional offset between the PET image and the CT image displayed in step S29.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. An image diagnosis apparatus **characterized by** comprising:
a detector (25) configured to detect gamma-rays emitted from inside an imaging region;
an acquisition unit (26) configured to acquire a plurality of first projection data sets associated with a plurality of projection angles via the detector (25);
a correction unit (83) configured to attenuation-correct the plurality of first projection data sets based on a first CT image associated with the imaging region to generate a plurality of second projection data sets associated with the plurality of projection angles; and
a calculation unit (85) configured to calculate an index based on the plurality of second projection data sets, the index being corresponding to a degree of positional offset between the imaging region at the time of acquisition of a plurality of third projection data sets associated with the first CT image and the imaging region at the time of acquisition of the plurality of first projection data sets.

2. The apparatus according to claim 1, **characterized in that** the calculation unit (85) is configured to calculate a plurality of integral values associated with the plurality of projection angles by integrating the plurality of second projection data sets, and calculate the index based on the plurality of integral values.

3. The apparatus according to claim 2, **characterized in that** the index changes in accordance with a degree of variation in the plurality of integral values.

4. The apparatus according to claim 2 or 3, **characterized in that** the index is one of a standard deviation and a variance of the plurality of integral values.

5. The apparatus according to any one of claims 1 to 4, **characterized by** further comprising
a PET image reconstruction unit (89) configured to reconstruct a first PET image based on the plurality of second projection data sets, and
a display unit (91) configured to superimpose and display the first PET image and the first CT image.

6. The apparatus according to any one of claims 1 to 5, **characterized by** further comprising
an X-ray tube (13) configured to generate X-rays,
an X-ray detector (14) configured to detect X-rays generated from the X-ray tube (13), and
a CT image reconstruction unit (81) configured to reconstruct the first CT image based on the plurality of third projection data sets originating from an output from the X-ray detector (14).

7. The apparatus according to claim 6,
**characterized in that**
the CT image reconstruction unit (81) is configured to reconstruct second CT images based on an output from the X-ray detector (14) if the index is not more than a threshold, the second CT images being associated with a slice position shifted to a predetermined position and a predetermined direction,
the correction unit (83) is configured to attenuation-correct the plurality of first projection data sets based on the second CT images to generate a plurality of fourth projection data sets respectively, the plurality of fourth projection data sets being associated with the plurality of projection angles, and
the calculation unit (85) is configured to calculate the index based on the plurality of fourth projection data sets.

8. The apparatus according to any one of claims 1 to 7, **characterized by** further comprising
a PET image reconstruction unit (89) configured to reconstruct a second PET image based on the plurality of third projection data sets if the index is not less than the threshold, and
a display unit (91) configured to superimpose and display the second PET image and the second CT image.

9. The apparatus according to any one of claims 1 to 8, **characterized by** further comprising a display unit (91) configured to display the index.

10. An image diagnosis method **characterized by** comprising:
causing a detector (25) to detect gamma-rays emitted from inside an imaging region;
causing an acquisition unit (26) to acquire a plurality of first projection data sets associated with a plurality of projection angles via the detector (25);
causing an attenuation correction unit (83) to attenuation-correct each of the plurality of first projection data sets based on a first CT image associated with the imaging region to generate a plurality of second projection data sets associated with the plurality of projection angles; and
causing a calculation unit (85) to calculate an index based on the plurality of second projection data sets, the index being corresponding to a degree of positional offset between the imaging region at the time of acquisition of a plurality of third projection data sets associated with the first CT image and the imaging region at the time of acquisition of the plurality of first projection data sets.

11. The method according to claim 10, **characterized in that** in causing a calculation unit (85) to calculate, each of the plurality of second projection data sets is integrated to calculate a plurality of integral values associated with the plurality of projection angles, and the index is calculated based on the plurality of integral values.

12. The method according to claim 10 or 11,
**characterized by** further comprising
causing a reconstruction unit (89) to reconstruct the first PET image based on the plurality of second projection data sets, and
causing a display unit (91) to superimpose and display the first PET image and the first CT image.

13. The method according to any one of claims 10 to 12,
**characterized by** further comprising
causing an X-ray tube (13) to generate X-rays,
causing an X-ray detector (14) to detect X-rays generated from the X-ray tube (13) and transmitted through the subject, and
causing a reconstruction unit (81) to reconstruct the first CT image based on an output from the X-ray detector (14).

14. The method according to claim 13,
**characterized by** further comprising
causing the reconstruction unit (81) to reconstruct second CT images based on an output from the X-ray detector (14) if the index is not more than a threshold, second CT images being associated with a slice position shifted to a predetermined position and a predetermined direction,
causing the attenuation correction unit (83) to attenuation-correct the plurality of first projection data sets based on the second CT images to generate a plurality of fourth projection data sets respectively, the plurality of fourth projection data sets being associated with the plurality of projection angles, and
causing the calculation unit (85) to calculate an index based on the plurality of fourth projection data sets.

15. The method according to claim 14, **characterized by** further comprising
causing the reconstruction unit (89) to reconstruct a second PET image based on the plurality of fourth projection data sets if the index is not less than the threshold, and
causing the display unit (91) to superimpose and display the second PET image and the second CT image.
